# EUROPEAN PATENT SPECIFICATION

(11) **EP 2 747 685 B1**
(45) Date of publication and mention of the grant of the patent: **17.04.2019**
(21) Application number: 12780832.7
(22) Date of filing: 12.09.2012
(51) Int. Cl.: A61B 17/70

(54) **LOW INVASIVE PERCUTANEOUS INTERSPINOUS SPACER**
GERING-INVASIVE PERKUTANE ZWISCHENWIRBEL-ABSTANDSHALTER
ESPACEUR INTERÉPINEUX PERCUTANÉ FAIBLEMENT INVASIF

(30) Priority: 12.09.2011 IT VI20110245
(43) Date of publication of application: 02.07.2014
(73) Proprietor: Medical Intellectual Property S.r.l., 00182 Roma (IT)
(72) Inventor: ALEXANDRE, Alberto, 31100 Treviso (IT)
(74) Representative: Maroscia, Antonio
(86) International application number: PCT/IB2012/054746
(87) International publication number: WO 2013/038349

(56) References cited:
- WO-A1-2010/141793
- WO-A2-2008/052071

## Description

### Field of the invention

The present invention is generally applicable to the field of medical devices for surgery and particularly relates to a low-invasive percutaneous interspinous spacer for implantation between two adjacent vertebral spinous processes of a patient to be treated.

### Background art

Interspinous spacers are known to be surgical devices designed for stable implantation into the human body at the lumbar spine, to treat certain diseases, such as lumbar stenosis.

Particularly, interspinous spacers have the purpose of maintaining the spinous processes of adjacent vertebrae appropriately spaced to prevent spinal nerve compression and stabilize the spine.

A multiplicity of interspinous spacers are known in the art, which generally have an expandable guide cannula designed for percutaneous insertion into the patient's body, allowing the passage of a dilating or "distracting" element which is designed to be located between the vertebral spinous processes to space them and maintain them in their proper position.

One of the main drawbacks of prior art spacers is that they require the availability of a relatively large number of distractors of different sizes for insertion of the one that has the same size as the interspinous space of the patient.

Therefore, the surgeon is required to measure the interspinous distance with the utmost accuracy, for proper selection of the right distractor.

Accordingly, such spacers require high skills of the surgeon, who may frequently incur measurement or evaluation errors that might affect proper conduction of the procedure or require trial-and-error approaches, involving patient discomfort.

In an attempt to at least partially obviate the above drawbacks, interspinous spacers have been developed, which have a spacing member designed to be progressively expanded upon insertion.

WO2009101539 discloses a spacer having an expandable spacing member, comprising a main body with two peripheral portions adapted to be spaced by means of a lever mechanism or a four-bar linkage.

US2009118833 discloses a spacer having a spacing member with an elastic outer wall and a hollow interior, designed to be filled with a controlled amount of liquid to reach the desired size.

Nevertheless, these solutions have shown to be poorly accurate, and unreliable with time.

WO2008/052071 discloses a low-invasive percutaneous insterspinous spacer having all features contained in the preamble of claim 1

### Disclosure of the invention

The object of the present invention is to overcome the above drawbacks, by providing an interspinous spacer that is highly efficient and relatively cost-effective.

A particular object is to provide an interspinous spacer that affords highly accurate adjustment of the interspinous distance, thereby providing high reliability with time.

Another object is to provide an interspinous spacer that allows adjustment of the interspinous distance to the right value by a single application, thereby reducing patient discomfort.

Yet another object is to provide an interspinous spacer that has high mechanical strength, in addition to ensuring biocompatibility, sterilizability and gamma radiation resistance.

A further object is to provide an instrument set for implantation of an interspinous spacer that is easy to handle and reduces spacer implantation times, thereby allowing easy adjustment of the interspinous distance.

These and other objects, as more clearly explained hereafter, are fulfilled by a low-invasive percutaneous interspinous spacer as defined in claim 1.

With this particular configuration, the expander element may be adjusted to the right size in gradual fashion and by a single application, without requiring trial and error approaches.

Advantageous embodiments of the invention are as defined in the dependent claims.

### Brief description of the drawings

Further features and advantages of the invention will be more readily apparent upon reading of the detailed description of preferred non-exclusive embodiments of a spacer according to the invention, which are shown as non-limiting examples with the help of the annexed figures, in which:
FIG. 1 is a schematic view of a spacer of the invention, inserted in an interspinous space;
FIGS. 2 and 3 are perspective views of an interspinous spacer of the invention in an operating configuration with the maximum transverse dimension;
FIG. 4 is a side view of the spacer of Figs. 2 and 3;
FIG. 5 is a perspective view of a fastening member of the spacer;
FIGS. 6 and 7 are perspective views of the spacer in two additional configurations, with the transverse dimension smaller than the maximum;
FIGS. 8 to 10 are front and side views of the guide element of the spacer;
FIGS. 11 to 14 are side views of expander elements of the spacer;
Fig. 15 shows a spacer of the invention, associated with equipment for implantation thereof.

### Detailed description of one preferred embodiment

Referring to the above figures, a low-invasive percutaneous interspinous spacer of the invention, generally designated by numeral 1, will be designed for use in surgical procedures for treating diseases that affect the lumbar spine, such as lumbar stenosis.

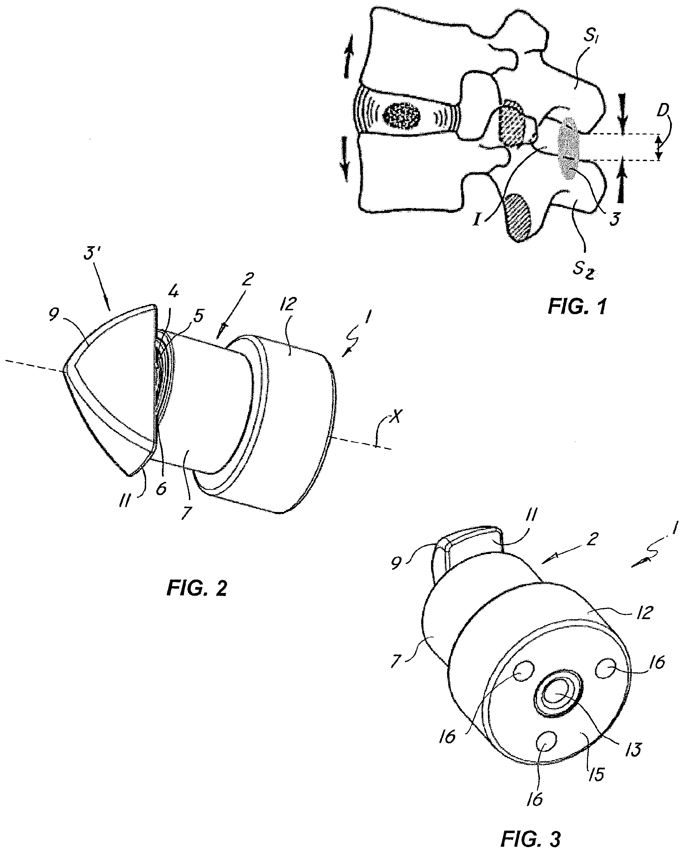

Particularly, the interspinous spacer 1 will be designed for implantation between two spinous processes S₁, S₂ of the vertebra to place them at an interspinous distance D to a maximum predetermined value, as schematically shown in Fig. 1.

The spacer 1 of the invention basically comprises expandable spacing means 2, adapted for insertion into the interspinous space I and having a maximum transverse dimension dₘₐₓ that can be adjusted to change the interspinous distance D to the desired value.

According to a peculiar feature of the invention, the spacing means 2 include a substantially longitudinal central guide member 3 adapted for percutaneous insertion into the interspinous space I.

Furthermore, the spacing means 2 include a plurality of substantially tubular expander elements or bushes 4, 5, 6, 7, having respective differentiated outer diameters φ_{e4,7}, progressively increasing from a minimum diameter φₑ₄ to a maximum diameter φₑ₇.

The expander elements 4-7 are adapted to be selectively and slideably fitted onto the cylindrical guide member 3 in mutually coaxial relationship from the tubular portion 4 having the minimum outer diameter φₑ₄ for gradual adjustment of the maximum transverse dimension dₘₐₓ of the spacing means 2 and progressively change the interspinous distance D.

The configuration as shown in Figs. 2 and 3 shows a spacer 1 having four substantially tubular expander elements 4, 5, 6, 7.

Furthermore, in this configuration, the tubular expander elements 4-7 are all fitted onto the guide element 3, to define a maximum transverse dimension dₘₐₓ configuration of the spacing means 2, thereby providing a distractor that will have a substantially cylindrical shape as a hole, and whose substantially constant outer diameter φₑ, will define the transverse dimension dₘₐₓ.

Nevertheless, it shall be understood that a different number of expander elements may be also provided.

Also, the number of expander elements fitted onto the guide element 3 and accordingly defining the distractor implanted in situ, may also be smaller than the maximum number of available expander elements, according to the desired transverse dimension, i.e. the interspinous distance D to be obtained.

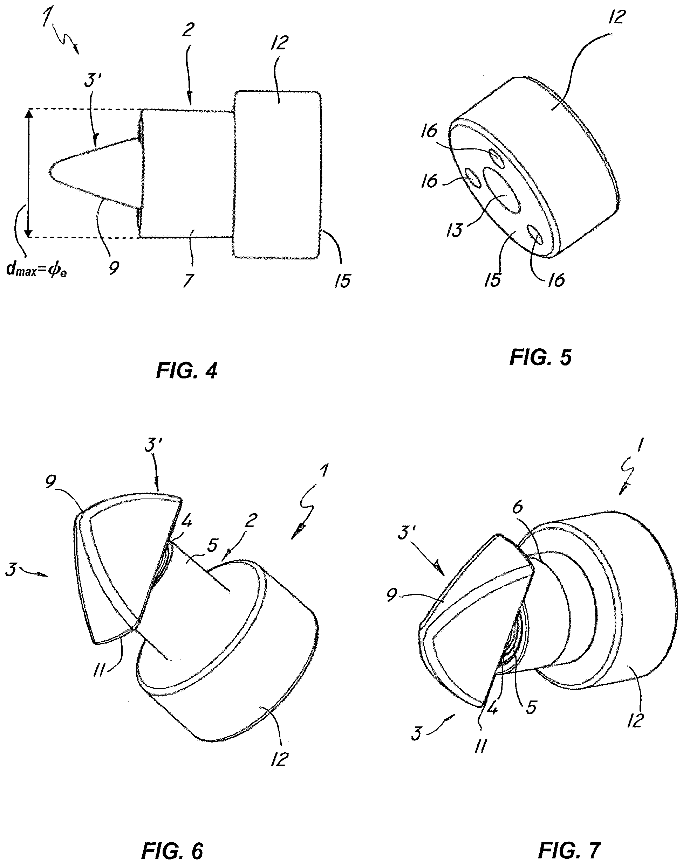

For instance, Fig. 6 shows a spacer 1 that only has the two innermost expander elements 4, 5 inserted therein, and Fig. 7 shows a spacer 1 in which the three innermost expander elements 4, 5, 6 are designed for implantation.

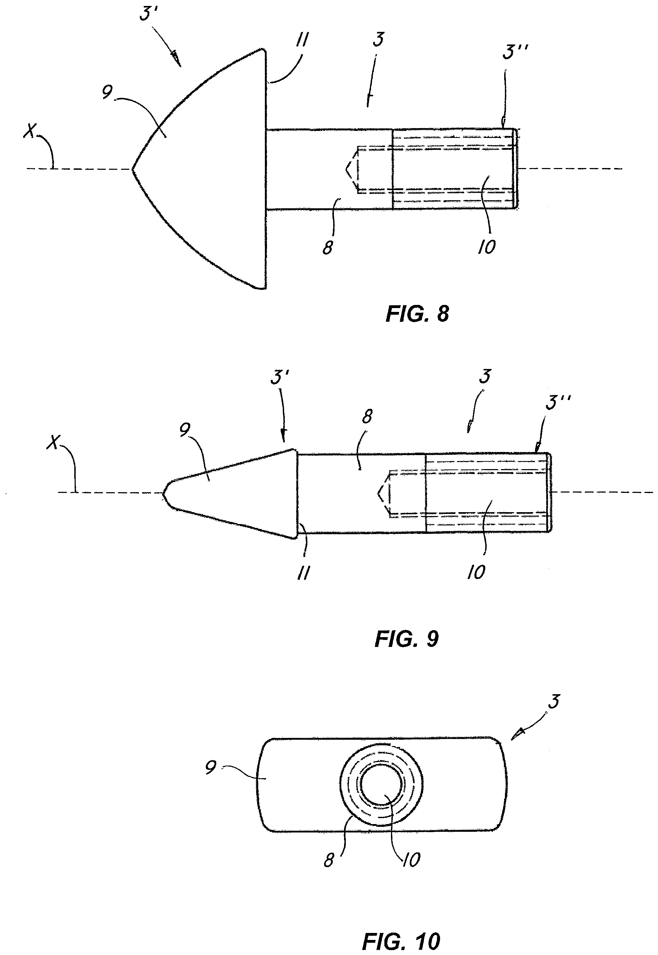

As more clearly shown in Figs. 8 to 10, the guide member 3 comprises a substantially elongate central section 8, coaxial with the expander elements 4-7, for support and axial guide thereof.

Furthermore, the guide member 3 has a first end 3' shaped with a substantially wedge-shaped front surface 9, for easier one-way insertion of the spacer 1 by translation thereof into the interspinous space I, when pressed by the operator or surgeon, and for anchorage thereof to the spinous processes S₁, S₂ of the vertebra to be spaced by rotation about its longitudinal axis X, thereby preventing spacer removal.

Furthermore, the guide member has, at its second proximal end 3", a substantially longitudinal and internally threaded cavity 10 for screw coupling to a driving stem, as described in greater detail below.

Conveniently, the first shaped end 3' of the guide member 3 has a substantially transverse flat rear surface 11 that defines an abutment for the axial sliding movement of the expander elements 4-7.

Advantageously, each expander element 4-7 may have an outer diameter φ_{e4,7} substantially matching the inner diameter φ_{i4,7} of the one of the other expander elements 4-7 that is designed to be fitted over it, to allow the expander elements 4-7 to lie one above the other.

Conveniently, such elements 4-7 may have substantially the same axial length l_{4,7}, thereby defining a substantially tubular cylindrical distractor 4 having substantially flat opposite transverse faces 2', 2".

Particularly, the transverse face 2' on the distal side, i.e. the side close to the first shaped end 3', abuts against the transverse surface 11 thereof.

As used herein, the terms "distal" and "proximal" shall be intended as a reference for the operator that uses the spacer and the instrument set, i.e. the distal side will be the one away from the operator during the procedure, and the proximal side will be the one close to the operator.

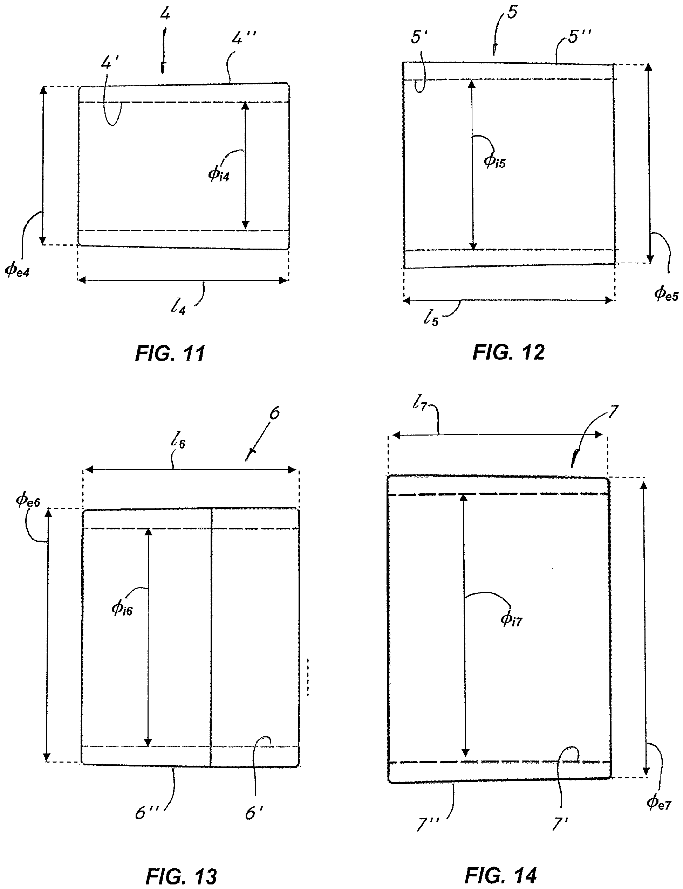

As shown in Figs. 11 to 14, the expander elements 4-7 have a slightly frustoconical shape. In the configuration of the invention, each expander element 4-7 has a substantially cylindrical inner surface 4'-7' and an at least partially frustoconical outer surface 4"-7".

For instance, as shown in Fig. 11, the innermost expander element 4, i.e. the one having the smallest radial dimension and designed to be directly placed on the guide member 3, may have an outer diameter φₑ₄ that slightly tapers toward the first end 3' of the guide member 3, for easier penetration of the interspinous space I. Conversely, its inner diameter φᵢ₄ is substantially constant.

The second 5 and fourth 7 expander elements, as shown in Figs. 12 and 14 respectively, may have respective outer diameters φₑ₅, φₑ₇ that slightly taper toward the second end 3" of the guide element 3, with respective inner diameters φᵢ₅, φᵢ₇ also substantially constant.

This will facilitate their sliding motion on the expander elements 4, 6 located directly therein, thereby ensuring stable interference fit thereof, once the expander elements 4-7 are implanted in situ.

Finally, Fig. 13 shows the third expander element 6 having a substantially constant inner diameter φᵢ₆, slightly higher than the maximum value of the outer diameter φₑ₅ of the second expander element 5.

Furthermore, the third expander element 6 has a distal section of its outer diameter φₑ₆ that increases toward the second end 3" of the guide element 3 and a proximal section having a substantially constant outer diameter φₑ₆.

These configurations allow mutual sliding of the expander elements 4-7, while affording stable fit thereof in situ, thereby allowing removal of the instruments required for implantation of the spacer 1, without incurring the risk of also removing the spacer 1 from the interspinous space I.

Conveniently, the spacer 1 may comprise a substantially annular fastening member 12, which is designed to be secured to the second end 3" of the guide member 3, substantially coaxial with the expander elements 4-7, to lock them in position against the abutment surface 1 and stabilize their position on the guide element 3.

The fastening member 12 has a substantially longitudinal and internally threaded central passage 13, for screw coupling thereof to the mating externally threaded proximal end 3" of the guide member 3.

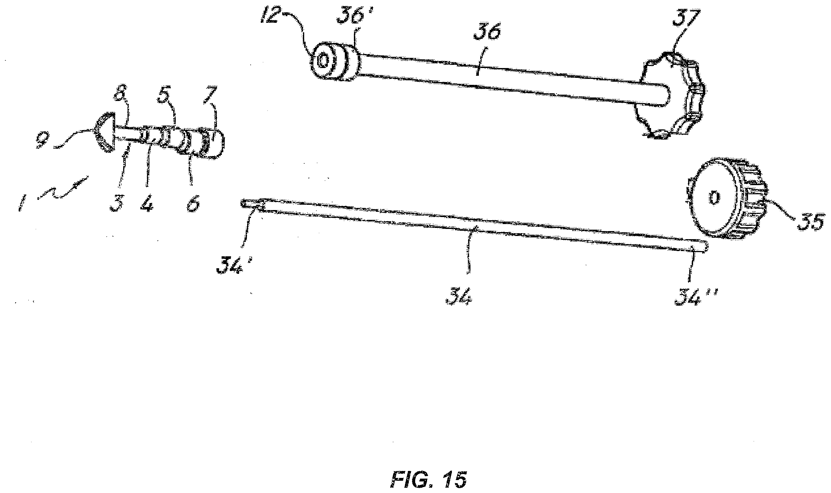

Furthermore, the fastening member 12 may have a plurality of blind holes 16, three in the exemplary configuration of the figures, on its proximal transverse face 15, which are designed to be engaged by a key element 36, as shown in Fig. 15 and described in greater detail below, for the operator to act upon the fastening member 12 and cause it to rotate, for screw coupling thereof to the guide member 3.

In a particularly advantageous aspect of the invention, all the elements of the spacer 1 may be made of one or more biocompatible polymeric materials.

Suitably, these materials may also be radiopaque, for detection thereof by X-rays and for continuous measurement of the interspinous distance D during the procedure.

In a particularly preferred configuration, polyether-ether-ketone (PEEK) or a similar material may be used, e.g. PEEK-1000 or another polymer derived from pure polyether-ether-ketone.

This material has a high mechanical and impact strength and allows sterilization by steam, dry air, ethylene oxide and gamma radiation.

Alternatively, silicone or similar polymeric resins, or even metal materials such as titanium or nickel-titanium, may be also used.

The spacer 1 will be introduced into the interspinous space I by first making an incision on the back of the patient at the surgical area, introducing the shaped end 3" of the guide member 3 with the front surface 9 in a substantially horizontal position and then inserting the expander elements 3 through an appropriate application instrument set, not shown.

The particular wedge shape of the front surface 9 will allow insertion thereof into the interspinous space I without tearing any tissue or causing trauma to the patient.

Once the guide member 3 is inserted, it will be rotated by about 90° through thrust means, as shown in Fig. 15, which include, in addition to the above mentioned key element 35, a longitudinal stem 34 having a distal end 34' integrally coupled to the second end 3" of the guide member 3 to rotate the latter about its axis X.

The distal end 34' of the stem 34 may be externally threaded to be screwed into the blind cavity 10 of the guide member 3, which is in turn internally threaded.

The stem 34 may be removably coupled, at its proximal end 34", to a driving disk 35 that will facilitate its movement and will allow rotation of the guide member 3 for securing the latter to the spinous processes S₁, S₂.

Then, the driving disk 35 that is screw-coupled to the stem 34 is operated to secure the spacer 1 to the spinous processes S₁, S₂ of the vertebra.

The key element 36 has a substantially elongate shape, with a distal end 36' shaped for engagement of the blind holes 16 of the fastening disk 12 and for screw coupling thereof to the second end 3" of the guide member 3. The key element 36 may be equipped with a ring nut 37 which is designed to be held by the operator for easier rotation thereof.

Also, a substantially elongate extractor 38 may be provided, which has an appropriately enlarged distal end 39 designed for interaction with one or more of the expander elements 4-7 during extraction of the application instruments after implantation.

Then, the selected thrust means will be used to cause gradual and selective forward movement of the expander elements 4-7 on the guide element 3 in the interspinous space I.

As soon as the optimal interspinous distance D is reached, the application instruments and the expander elements 4-7 that have not been inserted into the interspinous space, if any, are removed, and the driving disk 35 is unscrewed from the stem 34.

If the third 6 and/or fourth 7 expander elements have not been inserted, the extractor 38 may be used for extraction.

Then, the fastening member 12 coupled to the key element 36, which may have a tubular shape, will be inserted.

Thus, the fastening member 12 may be tightened on the second end 3" of the guide element 3 to stabilize the position of the expander elements 4-7.

Finally, both the stem 34 and the key element 36 will be removed, and leave the spacer 1 in situ.

The above disclosure clearly shows that the invention fulfills the intended objects and particularly provides an interspinous spacer that affords high accuracy and reliability and an instrument set allowing implantation thereof in a simple and quick manner, thereby reducing patient discomfort.

## Claims

1. A low-invasive percutaneous interspinous spacer, designed for insertion into the interspinous space (I) to change the distance between two adjacent spinous processes (S1, S2) to a predetermined maximum value, the spacer comprising spacing means (2) adapted for insertion into the interspinous space (I) and having an adjustable transverse dimension (dmax), wherein said spacing means (2) include a substantially cylindrical central guide member (3), which is adapted for insertion into the interspinous space (I), and a plurality of substantially tubular expander elements (4-7), having differentiated and progressively increasing outer diameters (φ_{e4,7}), said expander elements (4-7) being adapted to be selectively and slideably fitted onto said guide member (3) for progressively increasing the transverse dimension (dₘₐₓ) of said spacing means (2) to the predetermined maximum interspinous distance value (D), each of said expander elements (4-7) having an inner surface (4'-7') that is substantially cylindrical and an outer surface (4"-7");
**characterized in that** the substantially cylindrical inner surface (4'-7') of each of said expander elements (4-7) has an inner diameter (φ_{i4,7}) that is substantially constant, the outer surface (4"-7") of each of said expander elements (4-7) being at least partially frustoconical to allow mutual sliding of the expander elements (4-7) while affording stable fit thereof in situ.

2. Spacer as claimed in claim 1, **characterized in that** the innermost expander element (4) is designed to be directly placed on said guide member (3) and has an outer diameter (φₑ₄) that is slightly tapered toward a first longitudinal end (3') of the guide member (3).

3. Spacer as claimed in claim 2, **characterized in that** a second expander element (5) and the outermost expander element (7) have respective outer diameters (φₑ₅, φₑ₇) that are slightly tapered toward a second longitudinal end (3") of the guide member (3).

4. Spacer as claimed in claim 2, **characterized in that** a third expander element (6) has an inner diameter (φᵢ₆) that is slightly larger than the outer diameter (φₑ₅) of said second expander element (5).

5. Spacer as claimed in claim 2, **characterized in that** said third expander element (6) has a distal section with an outer diameter (φₑ₆) that increases toward the second end (3") of the guide element (3), and a proximal section having a substantially constant outer diameter (φₑ₆)

6. Spacer as claimed in claim 1, **characterized in that** said expander elements (4-7) substantially have the same axial lengths (14, 7) and are designed for external application to said central guide member (3) coaxial thereto.

7. Spacer as claimed in any preceding claim, **characterized in that** said guide member (3) defines a longitudinal axis (X) and its first end (3') has a substantially wedge-shaped front surface (9) to facilitate one-way insertion thereof into the interspinous space (I) and prevent removal thereof upon rotation about said longitudinal axis (X).

8. Spacer as claimed in claim 7, **characterized in that** said the first end (3') of said guide member (3) has a substantially flat and transverse rear surface (11) that defines an abutment surface for each of said expander elements (4-7).

9. Spacer as claimed in claim 1, **characterized in that** it comprises a substantially annular fastening member (12), which is adapted to be secured to the second end (3") of said central guide member (3) to lock said expander elements (4-7) in position.

10. Spacer as claimed in claim 9, **characterized in that** said fastening member (12) has a proximal transverse face (15) with a plurality of blind holes (16), which are designed to be engaged by a key element (36) to assist screw coupling thereof to the second end (3") of said guide member (3).

11. Spacer as claimed in any preceding claim, **characterized in that** said expander elements (4-7) are adapted to interact with external thrust means (34, 36) for facilitated insertion into the interspinous space (I).

12. Spacer as claimed in claim 11, **characterized in that** said second end (3") of said guide member (3) is threaded and is adapted to be integrally coupled to a matingly threaded longitudinal stem (34) of the thrust means for rotation about said longitudinal axis (X).

13. Spacer as claimed in any preceding claim, **characterized in that** it is made of a biocompatible and radiopaque material selected from the group comprising polymeric materials such as PEEK, silicone or similar polymeric resins and metal materials, such as titanium and titanium-nickel alloys.

## Patentansprüche

1. Ein niedriginvasiver perkutaner interspinalen Abstandshalter, der zum Einführen in den interspinalen Raum (I) vorgesehen ist, um den Abstand zwischen zwei benachbarten Dornfortsätzen (S1, S2) auf einen vorbestimmten Maximalwert zu ändern, wobei der Abstandshalter Abstandsmittel (2) umfasst, die zum Einführen in den interspinalen Zwischenraum (I) geeignet sind, wobei das Abstandmittel (2) eine einstellbare Querdimension (dₘₐₓ) aufweist, und ein im Wesentlichen zylindrisches zentrales Führungselement (3) umfass, das zum Einführen in den Zwischenraum (I) eingerichtet ist, und mehrere von im wesentlichen röhrenförmigen Expanderelementen (4-7) mit differenzierten und progressiv ansteigenden Außendurchmessern (φ_{e4,7}), wobei die Expanderelemente (4-7) selektiv und verschiebbar auf das Führungselement (3) zur progressiven Vergrößerung der Querdimension (dₘₐₓ) des Abstandsmittels (2) zu dem vorbestimmten maximalen Wert (D) des interspinalen Abstand aufgesteckt werden können, wobei jedes der Expanderelemente (4-7) eine innere, im Wesentlichen zylindrische, Oberfläche (4'-7') und eine äußere Oberfläche (4"-7") hat;
**dadurch gekennzeichnet, dass** die innere, im Wesentlichen zylindrische Oberfläche (4'-7') jedes der Expanderelemente (4-7) einen Innendurchmesser (φ_{i4,7}) hat, der im Wesentlichen konstant ist, wobei die Außenfläche (4"-7") jedes der Expanderelemente (4-7) zumindest teilweise kegelstumpfförmig ist, um ein gegenseitiges Gleiten der Expanderelemente (4-7) und gleichzeitig einen stabilen Sitz in situ zu ermöglichen.

2. Abstandshalter nach Anspruch 1, **dadurch gekennzeichnet, dass** das innerste Expanderelement (4) so ausgelegt ist, dass es direkt auf dem Führungselement (3) angeordnet werden kann und hat einen Außendurchmesser (φₑ₄), der sich nach einem ersten Längsende (3') des Führungselements (3) hin konisch leicht verdünnt.

3. Abstandshalter nach Anspruch 2, **dadurch gekennzeichnet, dass** ein zweites Expanderelement (5) und das äußerste Expanderelement (7) jeweilige Außendurchmesser (φₑ₅, φₑ₇) aufweisen, die sich nach einem zweiten Längsende (3") des Führungselements (3) hin konisch leicht verdünnen.

4. Abstandshalter nach Anspruch 2, **dadurch gekennzeichnet, dass** ein drittes Expanderelement (6) einen Innendurchmesser (φᵢ₆) hat, der etwas größer ist als der Außendurchmesser (φₑ₅) des zweiten Expanderelements (5).

5. Abstandshalter nach Anspruch 2, **dadurch gekennzeichnet, dass** das dritte Expanderelement (6) einen distalen Abschnitt mit einem Außendurchmesser (φₑ₆), der zum zweiten Ende (3") des Führungselements (3) hin zunimmt, und einen proximalen Abschnitt aufweist mit im Wesentlichen konstantem Außendurchmesser (φₑ₆)

6. Abstandshalter nach Anspruch 1, **dadurch gekennzeichnet, dass** die Expanderelemente (4-7) im Wesentlichen die gleichen axialen Längen (14, 7) aufweisen und zur externen koaxialen Anbringung an dem zentralen Führungselement (3) angeordnet werden können.

7. Abstandshalter nach irgendeinem der vorhergehenden Ansprüche, **dadurch gekennzeichnet, dass** das Führungselement (3) eine Längsachse (X) definiert und sein erstes Ende (3') eine im Wesentlichen keilförmige Vorderfläche (9) aufweist, um dessen einseitige Einführung in den interspinalen Raum (I) zu erleichtern und dessen Entfernung bei einer Drehung um die Längsachse (X) zu verhindern.

8. Abstandshalter nach Anspruch 7, **dadurch gekennzeichnet, dass** das erste Ende (3') des Führungselements (3) eine im Wesentlichen flache und quer verlaufende Rückfläche (11) aufweist, die eine Anschlagfläche für jedes der Expanderelemente (4-7) definiert.

9. Abstandshalter nach Anspruch 1, **dadurch gekennzeichnet, dass** er ein im Wesentlichen ringförmiges Befestigungselement (12) aufweist, das geeignet ist, an dem zweiten Ende (3") des zentralen Führungselements (3) befestigt zu werden, um die Expanderelemente (4-7) in ihrer Position zu verriegeln.

10. Abstandshalter nach Anspruch 9, **dadurch gekennzeichnet, dass** das Befestigungselement (12) eine proximale Querfläche (15) mit einer Vielzahl von Sacklöchern (16) aufweist, die so gestaltet sind, dass sie mit einem Schlüsselelement (36) in Eingriff stehen können, um ihre Schraubverbindung mit dem zweiten Ende (3") des Führungselements (3) zu erleichtern.

11. Abstandshalter nach irgendeinem der vorhergehenden Ansprüche, **dadurch gekennzeichnet, dass** die Expanderelemente (4-7) so gestaltet sind, dass sie mit äußeren Schubmitteln (34, 36) zusammenwirken können, um das Einführen in den interspinalen Raum (I) zu erleichtern.

12. Abstandshalter nach Anspruch 11, **dadurch gekennzeichnet, dass** das zweite Ende (3") des Führungselements (3) ein Gewinde aufweist und ist so ausgebildet, dass es an einen passenden, mit Gewinde versehenen Längsschaft (34) der Schubmittel zur Drehung um die Längsachse (X) vollständig gekuppelt werden kann.

13. Abstandshalter nach irgendeinem der vorhergehenden Ansprüche, **dadurch gekennzeichnet, dass** er aus einem biokompatiblen und strahlenundurchlässigen Material hergestellt ist, wobei das Material aus der Gruppe die Polymermaterialien wie PEEK, Silikon oder ähnliche Polymerharze umfasst, und
Metallmaterialien, wie Titan und Titan-Nickel Legierungen.

## Revendications

1. Une entretoise interépineuse percutanée mini-invasive, conçue pour être insérée dans l'espace interépineuse (I) afin de modifier la distance entre deux apophyses épineuses adjacentes (S1, S2) à une valeur maximale prédéterminée, l'entretoise comprenant des moyens d'écartement (2) conçus pour être insérés dans l'espace interépineux (I) et ayant une dimension transversale ajustable (dₘₐₓ), dans lequel lesdits moyens d'espacement (2) comprennent un élément de guidage central sensiblement cylindrique (3), qui est adapté pour être inséré dans l'espace interépineux (I), et une pluralité d'éléments d'expansion sensiblement tubulaires (4-7), ayant des diamètres extérieurs différenciés et augmentant progressivement (φ_{e4,7}), lesdits éléments d'élargissement (4-7) étant conçus pour être adaptés de manière à être montés de manière sélective et coulissante sur ledit élément de guidage (3) pour augmenter progressivement la dimension transversale (dₘₐₓ) dudit moyen d'espacement (2) par rapport à la valeur prédéterminée de la distance interépineuse (D), chacun desdits éléments d'expansion (4-7) ayant une surface intérieure (4'-7') qui est essentiellement cylindrique et une surface extérieure 4"-7");
**caractérisé en ce que** la surface intérieure sensiblement cylindrique (4'-7') de chacun desdits éléments d'extension (4-7) a un diamètre intérieur (φ_{i4,7}) sensiblement constant, la surface extérieure (4"-7") de chaque desdits éléments de dilatation (4-7) étant au moins partiellement tronconiques pour permettre un glissement mutuel des éléments de dilatation (4-7) tout en offrant un ajustement stable de ceux-ci in situ.

2. Entretoise selon la revendication 1, **caractérisée en ce que** l'élément extenseur le plus à l'intérieur (4) est conçu pour être placé directement sur ledit élément de guidage (3) et a un diamètre extérieur (φₑ₄) légèrement effilé en direction d'une première extrémité longitudinale (3') de l'élément de guidage (3).

3. Entretoise selon la revendication 2, **caractérisée en ce qu'**un deuxième élément de dilatation (5) et l'élément de dilatation le plus extérieur (7) ont des diamètres extérieurs respectifs (φₑ₅, φₑ₇) qui sont légèrement effilés vers une deuxième extrémité longitudinale (3") de membre de guidage (3).

4. Entretoise selon la revendication 2, **caractérisée en ce qu'**un troisième élément de dilatation (6) a un diamètre interne (φᵢ₆) légèrement supérieur au diamètre extérieur (φₑ₅) dudit deuxième élément d'expansion (5).

5. Entretoise selon la revendication 2, **caractérisée en ce que** ledit troisième élément d'expansion (6) a une section distale avec un diamètre extérieur (φₑ₆) qui augmente vers la deuxième extrémité (3") de l'élément de guidage (3) et une section proximale ayant un diamètre extérieur sensiblement constant (φₑ₆).

6. Entretoise selon la revendication 1, **caractérisée en ce que** lesdits éléments d'élargissement (4-7) ont sensiblement les mêmes longueurs axiales (14, 7) et sont conçus pour une application externe audit élément de guidage central (3) coaxial à celui-ci.

7. Entretoise selon l'une quelconque des revendications précédentes, **caractérisé en ce que** ledit élément de guidage (3) définit un axe longitudinal (X) et que sa première extrémité (3') présente une surface avant (9) sensiblement en forme de coin pour faciliter son insertion selon un seul sens dans l'espace interépineux (I) et empêcher son retrait lors d'une rotation autour dudit axe longitudinal (X).

8. Entretoise selon la revendication 7, **caractérisée en ce que** ladite première extrémité (3') dudit élément de guidage (3) présente une surface arrière sensiblement plate et transversale (11) qui définit une surface de butée pour chacun desdits éléments d'expansion (4-7).

9. Entretoise selon la revendication 1, **caractérisée en ce qu'**elle comprend un élément de fixation sensiblement annulaire (12), qui est adapté pour être fixé à la deuxième extrémité (3") dudit élément de guidage central (3) pour verrouiller en position lesdits éléments d'extension (4-7).

10. Entretoise selon la revendication 9, **caractérisée en ce que** ledit élément de fixation (12) présente une face transversale proximale (15) avec une pluralité de trous borgnes (16) conçus pour être engagés avec un élément de clé (36) pour assister le couplage à vis de celui-ci à la deuxième extrémité (3") dudit élément de guidage (3).

11. Entretoise selon l'une quelconque des revendications précédentes, **caractérisée en ce que** lesdits éléments d'extension (4-7) sont adaptés pour interagir avec des moyens de poussée externes (34, 36) pour faciliter l'insertion dans l'espace interépineux (I).

12. Entretoise selon la revendication 11, **caractérisée en ce que** ladite deuxième extrémité (3") dudit élément de guidage (3) est filetée et est adaptée pour être couplée de façon integrale au moyen d'un filetage correspondant à une tige longitudinale (34) des moyens de poussée pour rotation autour dudit axe longitudinal (X).

13. Entretoise selon l'une quelconque des revendications précédentes, **caractérisée en ce qu'**elle est constituée d'un matériau biocompatible et radio-opaque choisi dans le groupe comprenant les matériaux polymères tels que le PEEK, le silicone ou des résines polymères similaires et les matériaux métalliques, tels que titane et alliages titane-nickel.
